# EUROPÄISCHE PATENTSCHRIFT

(11) **EP 2 978 384 B1**
(45) Veröffentlichungstag und Bekanntmachung des Hinweises auf die Patenterteilung: **09.08.2017**
(21) Anmeldenummer: 14714623.7
(22) Anmeldetag: 21.03.2014
(51) Int. Cl.: A61C 1/08, A61C 13/00, A61B 5/00, A61B 34/10, A61C 5/42, A61C 5/44

(54) **VERFAHREN ZUR PLANUNG EINER WURZELBEHANDLUNG EINES PATIENTEN**
METHOD FOR PLANNING A ROOT TREATMENT OF A PATIENT
PROCÉDÉ DE PLANIFICATION DU TRAITEMENT RADICULAIRE D'UNE DENT D'UN PATIENT

(30) Priorität: 28.03.2013 DE 102013103209
(43) Veröffentlichungstag der Anmeldung: 03.02.2016
(73) Patentinhaber: Sicat GmbH & CO. KG, 53177 Bonn (DE)
(72) Erfinder: HEY, Joachim, 53639 Königswinter (DE); KUSCH, Jochen, 53343 Wachtenberg-Pesch (DE); ZOLLORSCH, Andreas, 53177 Bonn (DE)
(74) Vertreter: Braun-Dullaeus Pannen Patent- und Rechtsanwälte
(86) Internationale Anmeldenummer: PCT/EP2014/055712
(87) Internationale Veröffentlichungsnummer: WO 2014/154584

(56) Entgegenhaltungen:
- WO-A2-2011/101447
- DE-A1- 19 952 962

## Beschreibung

### Technisches Gebiet

Die Erfindung betrifft ein Verfahren zur Planung einer Wurzelkanalbehandlung eines Patienten unter Verwendung von dreidimensionalen Volumendaten vom zu bearbeitenden Zahn. Dabei kann eine Bohrhilfe verwendet werden.

Eine für das Verfahren typische Wurzelkanalbehandlung besteht darin, entzündetes Zahnmark zu entfernen, um den Zahn zu retten, selbst wenn er dann tot ist. Zur Entfernung des Zahnmarks muss der Wurzelkanal exakt freigelegt und keimfrei gemacht werden, um anschließend dicht verschlossen zu werden. Dazu muss zunächst der Eingang in den betroffenen Wurzelkanal gefunden werden. Danach wird mit einer Feile der Kanal freigelegt, wobei das Ziel darin besteht, den Kanal bis exakt zur Wurzelspitze freizulegen.

### Stand der Technik

Den Eingang zu einem Wurzelkanal zu finden, beruht maßgeblich auf der Erfahrung des behandelnden Zahnarztes. Im Backenzahnbereich ist es extrem schwierig, die Eingänge zu finden, da hier mehrere Wurzelkanäle vorliegen, die in ihrem Verlauf auch stark gekrümmt sein können. Die Anzahl der Wurzelkanäle und deren Verlauf sind anhand von Panoramaschichtaufnahmen oder Einzelzahnaufnahmen nicht immer eindeutig feststellbar.

Wird ein falscher Weg gefunden, der am eigentlichen Wurzelkanal vorbeigeht, so wird die entzündete Wurzel nicht entfernt und die sich anschließende Wurzelkanalbehandlung ist von vorneherein zum Scheitern verurteilt.

Zur Implantatplanung ist aus der DE 199 52 962 A1 und der WO2011101447 A2 bekannt, unter Verwendung von Oberflächendaten und von 3D-Daten eine Bohrhilfe zu erstellen.

Die Aufgabe der Erfindung besteht darin, die Möglichkeiten des Zahnarztes bei den Vorbereitungen einer Wurzelbehandlung zu verbessern.

### Darstellung der Erfindung

Die Erfindung betrifft ein Verfahren zur Planung einer Wurzelkanalbehandlung eines Patienten, wobei eine Kavität an einem zu behandelnden Zahn bereits präpariert ist, wobei eine Oberfläche der Kavität mittels eines optischen dreidimensionalen Messverfahrens vermessen wird und dabei dreidimensionale Messdaten der Kavität erzeugt werden. Anhand der erzeugten dreidimensionalen Messdaten wird dann ein 3D-Modell einer Führungsschablone geplant, die in ihren Abmessungen als Gegenstück zur präparierten Kavität ausgestaltet ist. Anhand von dreidimensionalen Volumendaten des zu behandelnden Zahns werden eine Lage und eine Orientierung mindestens eines Wurzelkanals bestimmt, wobei mindestens eine Führungsöffnung für ein Werkzeug zum Freilegen des Wurzelkanals geplant wird. Die Führungsöffnung ist dabei innerhalb der Führungsschablone so angeordnet, dass die Führungsöffnung auf einen Eintrittspunkt des Wurzelkanals und in eine Eintrittsrichtung des Wurzelkanals zeigt.

Nach der Bestimmung der Lage und der Orientierung der Wurzelkanäle relativ zu dem behandelnden Zahn werden auch die Eintrittsrichtung und der Eintrittspunkt der Wurzelkanäle definiert. Dadurch kann die Behandlung schneller und effizienter erfolgen, ohne dass Zahnsubstanz im Zahnbein und der Zahnkrone unnötig entfernt werden muss.

Der Eintrittspunkt kann dabei beispielsweise am mundhöhlenseitigen Ende des Wurzelkanals liegen.

Die hierzu erforderlichen dreidimensionalen Volumendaten über den Zahn und dessen innere Struktur können vorzugsweise mit einem dentalen Röntgengerät auf Basis der Cone-Beam-Technik gewonnen werden. Diese 3D-Daten werden benutzt, um mit Hilfe eines Computerprogramms die Wurzelkanäle zu identifizieren und die exakte Eintrittsrichtung und Länge des einzelnen Wurzelkanals zu definieren, z. B. in Bezug auf eine vorgegebene Ebene. Die Wurzelkanäle können dann im Programm markiert werden, so dass dadurch eine Wurzelkanalplanung möglich ist.

Die Lage des Eintrittspunktes und die Ausrichtung der Eintrittsrichtung jedes der Wurzelkanäle wird dabei in Relation zu den umgebenden anatomischen Strukturen, wie zu den Nachbarzähnen, bestimmt. Diese Referenzierung beziehungsweise Registrierung kann unter Verwendung von bestimmten markanten anatomischen Strukturen aus dem 3D-Datensatz, wie beispielsweise Strukturen auf den Okklusalflächen der benachbarten Zähne, oder unter Verwendung von speziellen Markierungen erfolgen, die auf einem Aufsatzteil bzw. auf einer Aufbissschiene angeordnet sein können. Dieses Aufsatzteil kann dabei während der Aufnahme zur Erzeugung des 3D-Datensatzes im Mundraum des Patienten auf dem zu behandelnden Zahn und/oder auf den Nachbarzähnen aufgesetzt sein. Dadurch wird eine eindeutige Lagebeziehung zwischen der Lage und der Ausrichtung der einzelnen Wurzelkanäle und den umgebenden anatomischen Strukturen aus dem 3D-Datensatz hergestellt.

Das optische dreidimensionale Messverfahren zur Erzeugung der dreidimensionalen Messdatenkavität kann beispielsweise ein Streifenprojektionsverfahren sein. Die Führungsschablone ist als Gegenstück zur präparierten Kavität ausgestaltet, so dass die Führungsschablone passgenau in die Kavität eingesetzt werden kann und eine präzise Bearbeitung des Wurzelkanals erlaubt.

Die Röntgendaten zur Bestimmung der Orientierung des Wurzelkanals relativ zum behandelnden Zahn können auch vor der Vermessung der Präparation erzeugt werden. Die Bestimmung des Eintrittspunkts und der Eintrittsrichtung des Wurzelkanals kann dabei anhand der Röntgendaten manuell durch den Benutzer oder computergestützt automatisch anhand von Mustererkennungsalgorithmen erfolgen.

Ein Vorteil dieses Verfahrens liegt darin, dass die Führungsschablone als Gegenstück zur präparierten Kavität geplant wird, die eine entsprechende Führungsöffnung aufweist, wobei die Führungsöffnung exakt auf den Eintrittspunkt und die Eintrittsrichtung des Wurzelkanals zeigt. Dadurch wird eine fehlerfreie Wurzelbehandlung ermöglicht, wobei der Wurzelkanal mittels des Werkzeugs zur Freilegung des Wurzelkanals exakt behandelt werden kann, ohne unnötig die umgebende Zahnsubstanz zu entfernen.

Die Herstellung der Führungsschablone kann beispielsweise mittels eines CAD/CAM-Systems vollautomatisch aus einem Rohling erfolgen.

Vorteilhafterweise kann bei der Wurzelbehandlung, welche nicht Teil der Erfindung ist, anhand der bekannten Abmessungen des einzusetzenden Werkzeugs und anhand einer Eindringtiefe des Werkzeugs in den Wurzelkanal ein Füllvolumen für ein Füllmaterial bestimmt werden. Dadurch wird verhindert, dass zuviel eingespritztes Füllmaterial an der Wurzelspitze austritt und dort Komplikationen hervorruft. Dadurch wird also eine Überfüllung des Wurzelkanals verhindert. Hierbei kann ein beliebiges Bearbeitungswerkzeug oder eine Kombination aus mehreren Bearbeitungswerkzeugen ausgewählt werden, um den Wurzelkanal möglichst gewebeschonend freizulegen. Dabei kann das Bearbeitungswerkzeug beispielsweise zylindrisch oder konisch zur Spitze zulaufend ausgestaltet sein. Dadurch kann das Volumen bereits vor der Bearbeitung des Zahns unter Berücksichtigung der Abmessungen des ausgewählten Bearbeitungswerkzeugs berechnet werden.

Falls der Wurzelkanal mit einem einzelnen Bearbeitungswerkzeug freigelegt wird, so hat der Bohrkanal eine zylindrische Form. Aus seiner Länge und dem Durchmesser lässt sich also dessen Volumen berechnen.

Falls der Wurzelkanal unter Verwendung mehrerer Bearbeitungswerkzeugen mit abnehmendem Durchmesser freigelegt wird, so hat der Bohrkanal eine sich stufenförmig zur Wurzelkanalspitze verengende Form. Aus der Summe der einzelnen Wegstrecken mit gleichem Durchmesser lässt sich dann ebenfalls das Volumen des Bohrkanals bestimmen. Falls der Wurzelkanal unter Verwendung eines konischen Bearbeitungswerkzeugs freigelegt wird, lässt sich das Volumen unter Verwendung der Abmessung des Bearbeitungswerkzeugs und der Eindringtiefe relativ zum Eintrittspunkt berechnen.

Vorteilhafterweise können zum Freilegen des Wurzelkanals mehrere Werkzeuge mit zunehmendem Durchmesser und abnehmender Eindringtiefe verwendet werden, so dass das Füllvolumen anhand der bekannten Abmessungen der einzelnen Werkzeuge und der geplanten Eindringtiefen für jedes der Werkzeuge berechnet wird.

Dadurch kann ein in der Dicke passendes Bearbeitungswerkzeug ausgewählt werden, üblicherweise ein Bohrer oder eine Feile. Die Dicke des Wurzelkanals kann sich zur Wurzelspitze hin verringern. Eine Möglichkeit der Behandlung ist, dass der Wurzelkanal seiner ganzen Länge nach mit einem breiten Bearbeitungswerkzeug freigelegt wird, das einen Durchmesser des Startpunktes des Wurzelkanals am Eintrittspunkt aufweist. Bei einer weiteren möglichen Behandlung des Wurzelkanals können weitere Bearbeitungswerkzeuge mit abnehmendem Durchmesser ausgewählt und nacheinander eingesetzt werden, um den sich verendenden Wurzelkanal vollständig Schritt für Schritt freizulegen, ohne das den

Wurzelkanal umgebende Zahnmaterial abzutragen.

Alternativ dazu kann auch ein einziges Bearbeitungswerkzeug verwendet werden, dass konisch zur Spitze hin geformt ist und in seinen Abmessungen den Abmessungen eines durchschnittlichen Wurzelkanals entspricht.

Der Vorteil eines solchen Wechsels liegt darin, dass der Wurzelkanal besonders schonend für das umgebende Zahngewebe freigelegt werden kann.

Durch dieses Verfahren wird also bereits in der Planungsphase die Länge und die Position des Wurzelkanals bestimmt und anschließend ein passendes Bearbeitungswerkzeug oder mehrere Bearbeitungswerkzeuge mit zunehmendem Durchmesser ausgewählt, um den Wurzelkanal besonders schonend freizulegen. Dabei kann bereits vor der Behandlung die Eindringtiefe relativ zum Eintrittspunkt für die einzelnen ausgewählten Bearbeitungswerkzeuge geplant werden.

Vorteilhafterweise kann die geplante Führungsschablone mittels einer Bearbeitungsmaschine hergestellt werden.

Die Herstellung der Führungsschablone kann beispielsweise vollautomatisch computergestützt mittels eines CAD/CAM-Systems erfolgen, wobei die Führungsschablone anhand des geplanten 3D-Modells aus einem Rohling vollautomatisch herausgeschliffen wird.

Vorteilhafterweise kann eine Stirnfläche der Führungsschablone als eine ebene Fläche geplant werden, die senkrecht zu einer Zahnachse angeordnet ist.

Dadurch kann die ebene Stirnfläche als ein Anschlag für das Bearbeitungswerkzeug verwendet werden.

Vorteilhafterweise kann eine Stirnfläche der Führungsschablone einer Okklusionsfläche des zu behandelnden Zahns entsprechen.

Die Stirnfläche kann der Okklusionsfläche des ursprünglichen Zahns entsprechen oder auch anhand der Messdaten der Nachbarzähne und der Gegenzähne neu geplant werden. Eine solche Führungsschablone kann nach der Wurzelbehandlung auch als ein Inlay verwendet werden, wobei lediglich der Führungskanal wieder verschlossen wird.

Vorteilhafterweise kann aus der Führungsschablone durch das Verschließen der Führungsöffnung ein Inlay hergestellt werden oder die Führungsschablone als Vorlage für die Herstellung eines Inlays verwendet wird.

Dadurch wird die Herstellung eines Inlays nach der Wurzelbehandlung erleichtert. Die Führungsöffnung kann mit einem geeigneten Material, wie Zahnzement, verschlossen werden.

Vorteilhafterweise kann die Herstellung der Führungsschablone vollautomatisch mittels eines CAD/CAM-Systems aus einem Rohling nach einem Bearbeitungsplan erfolgen.

Dabei kann in einem ersten Schritt das mittels einer CAD-Einheit geplante 3D-Modell der Führungsschablone in den Bearbeitungsplan übersetzt werden, der mehrere Maschinenbefehle enthält. Diese Maschinenbefehle werden dann im zweiten Schritt an eine CAM-Einheit, wie eine herkömmliche Bearbeitungsmaschine mit mehreren Fräswerkzeugen, übermittelt. Anschließend wird im dritten Schritt die geplante Bohrungsschablone vollautomatisch aus dem in die Bearbeitungsmaschine eingespannten Rohling herausgearbeitet.

Dadurch wird der Herstellungsprozess der Führungsschablone vereinfacht und beschleunigt. Weiter offenbart ist eine Führungsschablone für eine Wurzelkanalbehandlung eines Patienten, wobei die Führungsschablone mindestens eine Führungsöffnung aufweist. Die Führungsschablone weist eine Mantelfläche auf, die als Gegenstück zu einer präparierten Kavität eines zu behandelnden Zahns geformt ist, wobei die Führungsöffnung auf einen Eintrittspunkt und in eine Eintrittsrichtung mindestens eines Wurzelkanals des zu behandelnden Zahns zeigt.

Diese Führungsschablone hat den Vorteil, dass sie als Gegenstück zur präparierten Kavität geformt ist und dadurch eine eindeutige Positionierung relativ zum behandelnden Zahn ermöglicht. Dadurch können Positionierungsfehler wie an der Wurzelbehandlung verhindert werden.

Ein weiterer Vorteil dieser Führungsschablone besteht darin, dass die Wurzelbehandlung gewebeschonend durchgeführt werden kann, wobei das den Eintrittspunkt des Wurzelkanals umgebende Zahnmaterial nicht entfernt werden muss.

Vorteilhafterweise kann eine Stirnfläche der Führungsschablone als eine ebene Fläche ausgestaltet sein, die senkrecht zu einer Zahnachse des zu behandelnden Zahns angeordnet ist.

Dadurch kann die ebene Stirnfläche als Positionshilfe für den Benutzer und als Anschlagsfläche für das Werkzeug verwendet werden.

Vorteilhafterweise kann eine Stirnfläche der Führungsschablone als eine Okklusionsfläche des zu behandelnden Zahns ausgestaltet sein.

Dadurch kann aus der Führungsschablone mit der Okklusionsfläche auf einfache Art und Weise ein passendes inlay hergestellt werden, wobei lediglich die Führungsöffnung mit einem passenden Material, wie mit Zahnzement, verschlossen wird.

### Kurzbeschreibung der Zeichnungen

In der Zeichnung ist ein Ausführungsbeispiel der Erfindung dargestellt. Es zeigt:
- Fig. 1: eine Skizze zur Verdeutlichung des Verfahrens zur Planung einer Wurzelbehandlung;
- Fig. 2: eine Skizze zur Verdeutlichung der Durchführung der Wurzelbehandlung, welche nicht Teil der Erfindung ist, mittels eines speziellen Werkzeugs;
- Fig. 3: eine Skizze zur Verdeutlichung der Planung mittels eines Computers.

### Ausführungsbeispiel der Erfindung

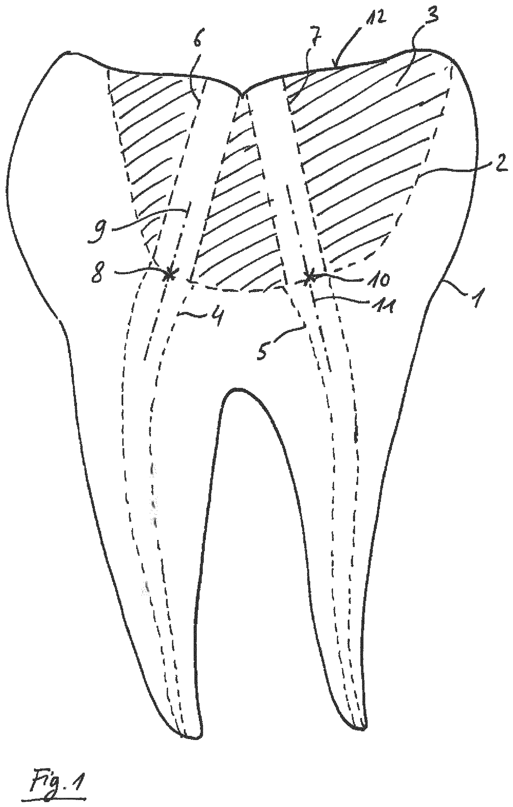

Die Fig. 1 zeigt eine Skizze zur Verdeutlichung des Verfahrens zur Planung einer Wurzelbehandlung. An einem zu behandelnden Zahn 1 ist bereits eine Kavität 2 präpariert. In einem ersten Verfahrensschritt wird diese Kavität 2 mittels eines optischen dreidimensionalen Messverfahrens vermessen und dabei dreidimensionale Messdaten der Kavität 2 erzeugt. Anhand dieser erzeugten Messdaten wird dann im zweiten Verfahrensschritt ein 3D-Modell einer Führungsschablone 3, die gestrichelt dargestellt ist, geplant, wobei die Führungsschablone 3 in ihren Abmessungen als Gegenstück zur Kavität 2 geformt ist. Anhand von Röntgendaten des zu behandelnden Zahns 1 wird die Lage und Orientierung eines ersten Wurzelkanals 4 und eines zweiten Wurzelkanals 5 bestimmt. Anschließend wird eine erste Führungsöffnung 6 und eine zweite Führungsöffnung 7 innerhalb der Führungsschablone 3 so geplant, dass die erste Führungsöffnung 6 auf einen ersten Eintrittspunkt 8 des ersten Wurzelkanals 4 in eine erste Eintrittsrichtung 9 zeigt und so dass die zweite Führungsöffnung 7 auf einen zweiten Eintrittspunkt 10 des zweiten Wurzelkanals 5 in eine zweite Eintrittsrichtung 11 zeigt. Nach der Planung des 3D-Modells wird die Führungsschablone 3 mittels einer CAM-Bearbeitungsmaschine hergestellt und, wie in Fig. 1 dargestellt, passgenau in die präparierte Kavität 2 eingesetzt. Anschließend wird die Wurzelbehandlung mittels eines zum Freilegen des Wurzelkanals geeigneten Werkzeugs durchgeführt, wobei das Werkzeug mittels der Führungsöffnungen 4 und 5 geführt wird. Nach der Durchführung der Wurzelbehandlung kann die Führungsschablone 3 auch zur Herstellung eines Inlays verwendet werden, wobei die Führungsöffnungen 4 und 5 mit einem geeigneten Material, wie mit Zahnzement, gefüllt werden. Ein solches Inlay kann dann passgenau in die Präparation eingesetzt werden. Die in Fig. 1 dargestellte Führungsschablone 3 weist eine Stirnfläche 12 auf, die als Okklusionsfläche des zu behandelnden Zahns 1 geformt ist. Die Stirnfläche 12 kann auch als eine ebene Fläche geformt sein.

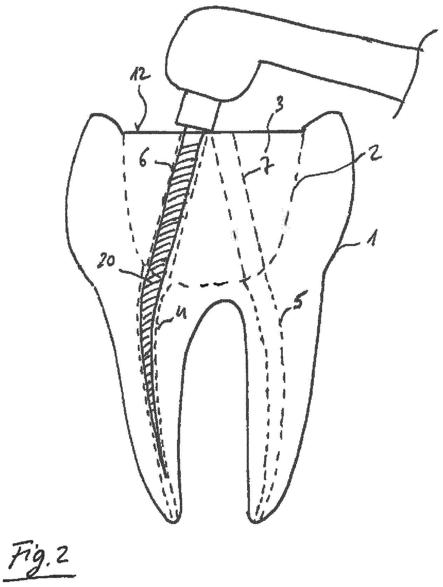

Die Fig. 2 zeigt eine Skizze zur Verdeutlichung der Durchführung der Wurzelbehandlung mittels der Führungsschablone 3. Zum Freilegen der Wurzelkanäle 4, 5 wird eine spezielles Werkzeug 20 verwendet, das zum vorliegenden Fall als eine konisch zulaufende flexible Feile ausgestaltet ist. Das Werkzeug 20 wird also durch die Führungsöffnungen 6 und 7 so geführt, dass es in die Wurzelkanäle 4, 5 positionsgenau eindringen kann. Die Stirnfläche 12, die im vorliegenden Fall als eine ebene Fläche ausführt ist, dient als ein Anschlag für das Werkzeug 20, der die Eindringtiefe des Werkzeugs 20 in den Wurzelkanal 4, 5 festlegt. Anhand der Abmessungen des einzusetzenden Werkzeugs 20 und anhand der Eindringtiefe des Werkzeugs in den Wurzelkanal 4, 5 kann ein Füllvolumen des Füllmaterials bestimmt werden. Dieses Füllmaterial wird nach dem Freilegen der Wurzelkanäle 4, 5 in die Wurzelkanäle bis zum Eintrittspunkt 8, 10 hineingespritzt. Die Bestimmung des Füllvolumens ist wesentlich, da ein zu geringes Füllvolumen zu einer unzureichenden Wurzelbehandlung im oberen Segment des Wurzelkanals und ein zu hohes Füllvolumen zu einem Überfüllen des Wurzelkanals 4, 5 führen kann. Falls das Füllmaterial an den spitzen Enden der Zahnwurzeln 4, 5 austritt, kann dies zu einer Schädigung der dort angeordneten Nervenbahnen führen. Zum Freilegen des Wurzelkanals 4, 5 können auch mehrere Werkzeuge mit zunehmenden Durchmesser und abnehmender Eindringtiefe verwendet werden, wobei die Werkzeuge konisch oder auch zylindrisch geformt sein können.

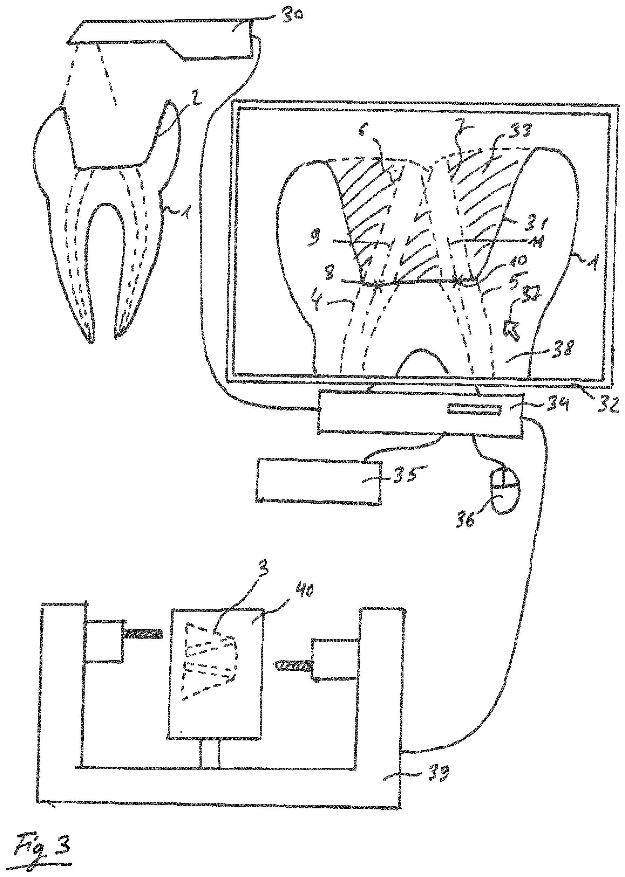

Die Fig. 3 zeigt eine Skizze zur Verdeutlichung des Verfahrens zur Planung der Wurzelbehandlung. In einem ersten Verfahrensschritt wird mittels einer Messkamera 30, die auf einem Streifenprojektionsverfahren basiert, die Kavität 2 des zu behandelnden Zahns 1 vermessen. Die erzeugten dreidimensionalen Messdaten 31 der Kavität 2 werden mittels einer Anzeigevorrichtung, wie eines Monitors 32 angezeigt. Anschließend wird anhand der erzeugten Messdaten 31 ein 3D-Modell 33 der Führungsschablone 3 geplant, die in den Abmessungen als Gegenstück zur Kavität 2 geformt ist. Bei der Planung werden die Führungsöffnungen 6 und 7 im 3D-Modell 33 so geplant, dass sie auf die Eintrittspunkte 8, 10 in die Eintrittsrichtungen 9, 11 der Wurzelkanäle 4, 5 zeigen. Die Planung erfolgt virtuell mittels eines Computers 34 an den die Eingabegeräte, wie die Tastatur 35 und Maus 36, angeschlossen sind. Zum Auswählen und zum Positionieren der Führungsöffnungen 6, 7 innerhalb des 3D-Modells 33 kann ein Cursor 37 verwendet werden. In Überlagerung mit den optischen Messdaten 31 werden Röntgen-Daten 38 des zu behandelnden Zahns 1 angezeigt, die eine Bestimmung der Lage und Orientierung der Wurzelkanäle 4, 5 erlauben. Die geplante Führungsschablone 3 wird dann nach dem 3D-Modell 33 mittels einer CAM-Bearbeitungsmaschine 39 aus einem Rohling 40 vollautomatisch herausgeschliffen. Die hergestellte Führungsschablone 3 kann dann immer wie in Fig. 1 gezeigt, in die Präparation 2 eingesetzt werden, um die geplante Wurzelbehandlung durchzuführen.

### Bezugszeichenliste

- 1: Zahn
- 2: Kavität/Präparation
- 3: Führungsschablone
- 4: erster Wurzelkanal
- 5: zweiter Wurzelkanal
- 6: erste Führungsöffnung
- 7: zweite Führungsöffnung
- 8: erster Eintrittspunkt
- 9: erste Eintrittsöffnung
- 10: zweiter Eintrittspunkt
- 11: zweite Eintrittsrichtung
- 12: Stirnfläche
- 20: Werkzeug
- 30: Messkamera
- 31: dreidimensionale Messdaten
- 32: Monitor
- 33: 3D-Modell
- 34: Computer
- 35: Tastatur
- 36: Maus
- 37: Cursor
- 38: Röntgen-Daten
- 39: CAM-Bearbeitungsmaschine
- 40: Rohling

## Patentansprüche

1. Verfahren zur Planung einer Wurzelkanalbehandlung eines Patienten, wobei eine Kavität (2) an einem zu behandelnden Zahn (1) bereits präpariert ist, wobei eine Oberfläche der Kavität (2) mittels eines optischen dreidimensionalen Messverfahrens vermessen wird und dabei dreidimensionale Messdaten (31) der Kavität (2) erzeugt werden,
**dadurch gekennzeichnet,**
**dass** anhand der erzeugten dreidimensionalen Messdaten (31) ein 3D-Modell (33) einer Führungsschablone (3) geplant wird, die in ihren Abmessungen als Gegenstück zur präparierten Kavität (2) ausgestaltet ist, wobei anhand von dreidimensionalen Volumendaten (38) des zu behandelnden Zahns (1) eine Lage und eine Orientierung mindestens eines Wurzelkanals (4, 5) bestimmt werden, wobei mindestens eine Führungsöffnung (6, 7) für ein Werkzeug (20) zum Freilegen des Wurzelkanals (4, 5) geplant wird, wobei die Führungsöffnung (6, 7) innerhalb der Führungsschablone (3) so angeordnet ist, dass die Führungsöffnung (6, 7) auf einen Eintrittspunkt (8, 10) des Wurzelkanals (4, 5) und in eine Eintrittsrichtung (9, 11) des Wurzelkanals (4, 5) zeigt.

2. Verfahren nach Anspruch 1, **dadurch gekennzeichnet, dass** die geplante Führungsschablone (3) mittels einer Bearbeitungsmaschine (39) hergestellt wird.

3. Verfahren nach einem der Ansprüche 1 bis 2, **dadurch gekennzeichnet, dass** eine Stirnfläche (12) der Führungsschablone (3) als eine ebene Fläche geplant wird, die senkrecht zu einer Zahnachse angeordnet ist.

4. Verfahren nach einem der Ansprüche 1 bis 2, **dadurch gekennzeichnet, dass** eine Stirnfläche (12) der Führungsschablone (3) einer Okklusionsfläche des zu behandelnden Zahns (1) entspricht.

5. Verfahren nach einem der Ansprüche 1 bis 4, **dadurch gekennzeichnet, dass** aus der Führungsschablone (3) durch das Verschließen der Führungsöffnung (6, 7) ein Inlay hergestellt wird oder die Führungsschablone (3) als Vorlage für die Herstellung eines Inlays verwendet wird.

6. Verfahren nach einem der Ansprüche 1 bis 5, **dadurch gekennzeichnet, dass** die Herstellung der Führungsschablone (3) vollautomatisch mittels eines CAD/CAM-Systems aus einem Rohling nach einem Bearbeitungsplan erfolgt.

## Claims

1. A method for planning a root canal treatment for a patient, wherein a cavity (2) on a tooth (1) to be treated has already been prepared, a surface of the cavity (2) being measured by means of an optical three-dimensional measurement method and three-dimensional measured data (31) on the cavity (2) being generated,
**characterized in that**
a 3-D model (33) of a guide template (3) is planned on the basis of the three-dimensional measurement data (31) thereby generated, the dimensions of the guide template (3) being designed as a counterpart to the prepared cavity (2), wherein a position and an orientation of at least one root canal (4, 5) are determined on the basis of three-dimensional volume data (38) on the tooth (1) to be treated, wherein at least one guide opening (6, 7) for a tool (20) for exposing the root canal (4, 5) is planned, wherein the guide opening (6, 7) is disposed inside the guide template (3), so that the guide opening (6, 7) points to an entrance point (8, 10) of the root canal (4, 5) and to a direction of an entrance (9, 11) to the root canal (4, 5).

2. The method according to claim 1,
**characterized in that**
the planned guide template (3) is created by means of a processing machine (39).

3. The method according to any one of claims 1 to 2, **characterized in that**
an end face (12) of the guide template (3) is designed as a flat surface disposed at a right angle to an axis of a tooth.

4. The method according to any one of claims 1 to 2,
**characterized in that**
an end face (12) of the guide template (3) corresponds to an occlusive surface of the tooth (1) to be treated.

5. The method according to any one of claims 1 to 4,
**characterized in that**
an inlay is produced from the guide template (3) by closing the guide opening (6, 7), or the guide template (3) is used as a model for producing an inlay.

6. The method according to any one of claims 1 to 5,
**characterized in that**
the production of the guide template (3) takes place in a fully automatic process by means of a CAD/CAM system starting with a blank according to a processing design.

## Revendications

1. Procédé de planification d'un traitement de canal radiculaire d'un patient, une cavité (2) étant déjà préparée sur une dent à traiter (1), une surface de la cavité (2) étant mesurée au moyen d'un procédé de mesure optique tridimensionnel et des données de mesure tridimensionnelles (31) de la cavité (2) étant produites à cet effet,
**caractérisé en ce**
**qu'**un modèle tridimensionnel (3-D) (33) d'un gabarit de guidage (3) est planifié à l'aide des données de mesure tridimensionnelles produites (31), qui est constitué dans ses dimensions comme une contre-pièce pour la cavité préparée (2), une position et une orientation d'au moins un canal radiculaire (4, 5) étant déterminées à l'aide des données de volume tridimensionnelles (38) de la dent à traiter (1), au moins une ouverture de guidage (6, 7) pour un outil (20) étant planifiée pour découvrir le canal radiculaire (4, 5), l'ouverture de guidage (6, 7) à l'intérieur du gabarit de guidage (3) étant disposée de telle manière que l'ouverture de guidage (6, 7) est dirigée vers un point d'entrée (8, 10) du canal radiculaire (4, 5) et dans une direction d'entrée (9, 11) du canal radiculaire (4, 5).

2. Procédé selon la revendication 1,
**caractérisé en ce que**
le gabarit de guidage planifié (3) est fabriqué au moyen d'une machine d'usinage (39).

3. Procédé selon l'une quelconque des revendications 1 à 2,
**caractérisé en ce**
**qu'**une face avant (12) du gabarit de guidage (3) est planifiée comme une surface plane qui est disposée perpendiculairement à un axe de dent.

4. Procédé selon l'une quelconque des revendications 1 à 2,
**caractérisée en ce**
**qu'**une face avant (12) du gabarit de guidage (3) correspond à une surface d'occlusion de la dent à traiter (1).

5. Procédé selon l'une quelconque des revendications 1 à 4,
**caractérisé en ce**
**qu'**un inlay est fabriqué à partir du gabarit de guidage (3) par l'occlusion de l'ouverture de guidage (6, 7) ou le gabarit de guidage (3) est utilisé comme modèle pour la fabrication d'un inlay.

6. Procédé selon l'une quelconque des revendications 1 à 5,
**caractérisé en ce que**
la fabrication du gabarit de guidage (3) a lieu selon un programme d'usinage entièrement automatique au moyen d'un système de CAO/FAO à partir d'une ébauche.
